# EUROPEAN PATENT APPLICATION

(11) **EP 4 821 462 A1**
(43) Date of publication of application: **14.10.2026**
(21) Application number: 25290015.4
(22) Date of filing: 08.04.2025
(51) Int. Cl.: A61F 2/14, A61B 17/3205

(54) **DEVICE AND METHOD FOR CUTTING OPHTHALMIC GRAFT MATERIAL**

(71) Applicant: Vision Innovation Solutions Pty Ltd, Herston, QLD 4006 (AU); Gatinel, Damien, 75006 Paris (FR)
(72) Inventor: Trinh, Tanya, Mosman New South Wales, 2088 (AU); Gatinel, Damien, 75006 Paris (FR)
(74) Representative: Calysta NV

(57) **Abstract**

Device (1) for cutting an ophthalmic graft material (2), the device (1) comprising a cutting tool (10) for cutting the ophthalmic graft material (2), the device optionally comprising a graft holder (12), wherein the cutting tool (10) is configured to cut the ophthalmic graft material (2) such to extract a spiral shaped strip from the ophthalmic graft material (2).

## Description

### Technical Field

The present invention relates to devices and methods used in ophthalmic procedures and particularly to devices and methods for cutting ophthalmic graft material.

### Prior art

Corneal ectatic disorders, such as keratoconus, pellucid marginal degeneration, and post-refractive ectasia, are characterized by irregularities in corneal morphology that impair biomechanical stability and visual acuity. These disorders often require surgical interventions of the cornea to improve corneal biomechanics, such as keratoplasty. Keratoplasty involves the transplantation of a donor corneal tissue to replace damaged or diseased corneal layers.

Another type of surgical intervention to regularize corneal shape involves the implantation of intrastromal ring segments. Synthetic intrastromal corneal ring segments (ICRS) are arc-shaped implants, typically made of polymethylmethacrylate (PMMA), which are inserted in incisions created in the peripheral cornea stroma. One advantage of ICRS is their independence from donor availability. However, synthetic ICRS lack individualized customization which hinders their ability to adapt to the unique morphology of a patient's cornea and to treat advanced ectatic cases. They are also costly and extrusion and infection are not infrequent side effects of these procedures.

Recent advancements, such as customized corneal allogeneic intrastromal ring segments (CAIRS), have emerged as promising solutions for the treatment of Corneal ectatic disorders. CAIRS segments are cut out from allogenic corneal tissue and offer several advantages compared to synthetic ICRS, such as the ability to create custom-made grafts, superior biocompatibility due to their allogenic origin, and optical properties more closely resembling the rest of the cornea. Despite their advantages, some barriers hinder the broader adoption of CAIRS. Commercially available CAIRS segments exist, but are expensive and, although theoretically distributable across several countries, their cost renders them inaccessible in many regions. Moreover, precision cutting of the corneal tissue for customisation is crucial for obtaining CAIRS segments tailored to the specific morphology of the host cornea, which is less amenable in commercially available CAIRS segments due to the semi-rigid nature of the tissue compared to freshly prepared corneal transplant tissue.

Known surgical instruments for cutting corneal tissue, such as standard circular corneal trephines or corneal punches, are relatively affordable but lack the precision required to create the intricate shapes necessary for CAIRS. On the other hand, advanced cutting technologies, such as femtosecond lasers allow for customization of CAIRS segments, but are prohibitively expensive and not widely available, particularly in resource-limited settings.

The preceding discussion of the prior art is intended to facilitate an understanding of the present invention only. The discussion is not an acknowledgement or admission that any of the material referred to is or was part of the common general knowledge as at the priority date of the application.

### Brief summary of the invention

It is an object of the invention to overcome the disadvantages of the prior art, or at least provide an alternative to the prior art.

It is, in particular, an object of the invention to enable a simple and low-cost preparation of customizable corneal segments. It is also an object of the invention to reduce the dependency on imported corneal segments and facilitate the use of locally sourced or bioengineered tissues.

According to the invention, this object is solved by a device for cutting an ophthalmic graft material according to the first claim, the device comprising a cutting tool for cutting the ophthalmic graft material, wherein the cutting tool is configured to cut the ophthalmic graft material such to extract an even width, spiral shaped strip from the ophthalmic graft material.

The invention advantageously allows to obtain a strip which is significantly longer than those produced by traditional methods from an equivalent surface area of ophthalmic graft material. For example, in the case of an allogenic graft where the donor cornea is the ophthalmic graft material from which the strip is cut, the strip length obtained with the present invention can exceed the external circumference of the donor cornea's surface. With the prior art, the donor cornea surface is normally a circle and the strip length was limited to the circumference of the circle. With the present invention, the strip length can become longer than the circumference of the circle. Having a longer and continuous strip enables segmentation of the strip into multiple segments of customizable lengths, which can e.g. be tailored to the specific requirements of the host or the surgical procedures. The prior art strips of the circumference were often cut to obtain a first strip and the remaining second strip was often too short for use in the same or second patient case. Thus, the current invention allows due to the significant increase of strip length obtained from one donor to obtain several strips for implantation in multiple eyes and potentially even into multiple patients. This reduces the donor material waste drastically and makes these procedures much more accessible. Therefore, it is possible to increase the degree of versatility and customizability of ophthalmic surgery applications relying on excised ophthalmic graft tissues, such as CAIRS. Moreover, a spiral shaped strip allows to cover a larger portion of the ophthalmic graft material surface compared to the solutions of the prior art, thereby increasing the amount of ophthalmic graft material that can be recovered as part of the strip and reduce the amount of ophthalmic graft material that is unused or discarded. The more efficient recovery of material is particularly advantageous when the ophthalmic graft material is donor-derived, due to the significant imbalance between the demand for donor corneas and the limited supply. It promotes the more ethical usage of transplant tissue by minimizing wastage. Overall, the present invention enables a more efficient approach to excise an ophthalmic graft material, which increases versatility, reduces waste and introduces the possibility of having multiple hosts benefiting from a single donor graft.

According to the invention, this object is also solved by a device for cutting an ophthalmic graft material, the device comprising a cutting tool for cutting the ophthalmic graft material, wherein the cutting tool is configured to cut a strip of ophthalmic graft material, , wherein the cutting path is formed such that the strip has a length that is greater than the exterior perimeter of the smallest circle formed around the cutting path in the ophthalmic graft material, preferably a spiral.

According to the invention, this object is also solved by a device for cutting corneal tissue, the device comprising a cutting tool for cutting the corneal tissue, wherein the cutting tool is configured to cut a strip of corneal tissue, wherein the cutting path is formed such that the strip has a length that is greater than the exterior perimeter of the corneal tissue, preferably a spiral.

According to a second aspect, the invention further relates to a method for excising an ophthalmic graft material using the device as described above, the method comprising a step of cutting by means of the cutting tool a strip of ophthalmic graft material, the strip being a spiral shaped strip.

According to the invention, this object is also solved by a computer program comprising instructions configured, when executed on a processor (such as a femtosecond laser), to perform the following steps: receiving data of one or more planned strips of ophthalmic graft material (e.g. a donor cornea) for implantation in the eye or the eyes of one or more receiving patients; computing for each of the one or more planned strips which part of a spiral shaped strip of ophthalmic graft material can be used.

The dependent claims refer to further optional embodiments of the invention.

In embodiments, the cutting tool is configured to cut the ophthalmic graft material such to extract the spiral shaped strip from ophthalmic graft material. A spiral shaped strip allows the recovery of a long strip of ophthalmic graft material. It also ensures smooth transitions between adjacent sub-portions of the strip due to the progressive increase of the spiral radius. It is therefore possible to obtain, from a single strip, a large number of strip segments and/or strip segments of extended length. The spiral shape also enables an efficient recovery of material by covering a large surface area of the ophthalmic graft material. For example, the first end of the spiral may be in a central region of a donor cornea, and the spiral shaped strip may extend towards a peripheral region of the donor cornea. This allows to further increase the surface area of the ophthalmic graft material that can be recovered as part of the strip.

In embodiments, the strip has a constant width. The width of the strip segment maintains a constant width from one end of the spiral to the other.

In embodiments, the strip may have a varying width. The width may be adjusted according to planning applications and may vary along its width along the length of the strip.

In embodiments, the cutting tool is a laser with a functionality to automatically cut the spiral shaped strip out of the ophthalmic graft material. For example, the laser may be a femtosecond laser.

In embodiments, the cutting tool is a blade having a spiral shape shape. The blade is preferably made of a material that is biocompatible and has sharp cutting properties. The blade material may be a material capable of withstanding sterilization. For example, blade may be made of metal, e.g. stainless steel. The blade, preferably the spiral shaped blade may have a diameter comprised between 2 mm and 18 mm, preferably between 5 mm and 15 mm. For adults, the blade would have normally a diameter between 10 and 14 mm, preferably between 11 and 13 mm corresponding to the diameter of the cornea.

These diameters are particularly suited for cutting ophthalmic graft materials. By "diameter of the blade", it is meant the diameter of the smallest circle that fully circumscribes the blade. For example, this diameter may be equal to the diameter of the largest turn of the spiral in a spiral shaped blade.

The blade is thin, i.e. it has a small thickness. This allows to perform precise cuts and reduce deformation of the ophthalmic graft material surrounding the cut.

The blade may have a bevelled cutting edge. In this case, the cutting edge may be bevelled on one side of the blade or on both sides of the blade.

In embodiments, the device comprises a blade holder having a proximal portion and a distal portion, and the blade extends outwardly from the distal portion of the blade holder.

The blade holder is configured to be held by a user (e.g. surgeon, eye bank personnel) or a robotic system.

The blade holder may comprise one or more features for improving ergonomics. For example, the blade holder may comprise a handle or inward-facing lobes designed to accommodate fingers during use.

The blade holder may utilize stabilization features such as a suction tube and syringe to allow the ophthalmic graft tissue or donor cornea to fixedly centrate and not move during the cutting process.

The blade holder may comprise one or more features for ensuring stable gripping. For example, the blade holder may comprise a textured surface e.g. having a surface rugosity and/or a pattern such as a repetition of protrusions or grooves. The blade holder may assume a vertical curvature at a gradient similar to that of a corneal curvature, and the corresponding blade may also assume a vertical curvature at a gradient similar to that of a corneal curvature.

The blade holder may be integrally formed with or fixedly attached to the blade holder.

Alternatively, the blade holder and the blade may be separate.

For example, the blade holder may be configured to hold various blade shapes and/or blade size, and the blade is an interchangeable blade which can be fitted in the blade holder.

The blade holder may be made of metal (e.g. aluminum) and/or plastic.

In embodiments, the blade may be retractably or movably mounted on the distal portion of the blade holder. The blade may be configured to move/retract with respect to the blade holder between a retracted position and an extended position. In this case, the device may include a retraction mechanism for moving the blade relative to the blade holder (between the retracted position and the extended position). The device is preferably positioned over the ophthalmic graft material and the retraction mechanism is activated to move the blade into the extended position in which the ophthalmic graft material is cut out. Preferably, after cutting out the ophthalmic graft material, the blade retracts back into the retracted position.

The retraction mechanism may be, for example, a push mechanism, configured such that a pushing force applied on the push mechanism extends the blade outward from the blade holder and a release of the pushing force or a pulling force applied on the push mechanism retracts (at least partially) the blade within the blade holder.

These retraction mechanisms allow to adjust the position of the blade relative to the blade holder and ensure safe storage of the device when the blade is not in use.

The retraction mechanism can have a cutting depth limitation mechanism for limiting the cutting depth of the blade. The cutting depth of the blade could be configurable.

In embodiments, the device further comprises a graft holder for supporting the ophthalmic graft material.

The graft holder allows to maintain the ophthalmic graft material in a stable position during use. The graft holder is made of a biocompatible material suitable for contact an ophthalmic graft material. The graft holder material may be a material capable of withstanding sterilization. For example, the graft holder may be made of metal and/or plastic (e.g. polycarbonate, polytetrafluoroethylene).

The graft holder may comprise a concave surface. For example, the curved concave surface may be hemispherical or bowl-shaped, protruding inwardly in or outwardly from the graft holder. These surfaces better conform to the natural curvature of a donor cornea.

The graft holder may comprise means to ensure its stability on a planar working surface such as a bench or table.

The graft holder may be at least partially optical transparent, such to facilitate real-time visualization of the cutting process.

The device may further comprise a stabilization mechanism for securing the ophthalmic graft material on the graft holder during use. For example, the stabilization mechanism may be a suction mechanism. The graft holder may comprise an interior lumen (e.g. comprising one or more holes, channels, and/or chambers) and the suction mechanism may be configured to apply a negative pressure to the lumen to facilitate contact between the ophthalmic graft material and the graft holder.

For example, the lumen may be fluidly connected with one or more first openings located on a first surface of the graft holder that is configured to receive the ophthalmic graft material. This first surface can e.g. be the surface of the graft holder that faces upward when the device is in its in-use configuration. One or more second opening may be positioned on another surface of the graft holder, such as a lateral or bottom surface when the device is in its in-use configuration. The second opening may be configured to be connectable to a vacuum source (e.g. a pump or a syringe).

In embodiments, the cutting tool may be configured to cooperate with the graft holder during use.

For example, the graft holder and/or the blade holder may comprise alignment markers (e.g. visual indicia) to assist a user (e.g. surgeon, eye bank personnel) in orienting the ophthalmic graft material relative to the cutting tool.

For example, the device may comprise an alignment mechanism comprising an alignment element provided on the blade holder, and a cooperating alignment element provided on the graft holder. For example, the alignment element on the blade holder may comprise one or more rods protruding from the distal portion of the blade holder, and the cooperating alignment element may comprise one or some recesses on the graft holder, each recess being configured to receive a corresponding protruding rod.

In embodiments, the device comprises a suction mechanism on the distal portion of the blade holder for positioning the blade holder with its distal portion on the ophthalmic graft

In embodiments, the device is a corneal trephine or a corneal punch. In one embodiment, the computer program is configured to calculate the correct length of the part the spiral shaped strip of ophthalmic graft material based on the length of the corresponding strip for the receiving patient.

In one embodiment, the computer program is configured to select the size and/or type of spiral used for cutting the ophthalmic graft material based on the data of the one or more planned strips.

In one embodiment, the computer program is configured to select different cutting depths of the spiral shaped strip based on the different thicknesses of the planned strips.

In embodiments, the method comprises, before cutting the strip of ophthalmic graft material, a step of creating a negative pressure between the cutting tool and the ophthalmic graft material.

In one embodiment, the computer program generates control signals for the laser to cut the spiral shaped strip.

In one embodiment, the computer program is configured to specifying precise coordinates for the spiral path (in depth and/or in the cutting plane).

In one embodiment, the computer program is configured to perform an automatized custom spiral planning.

In one embodiment, the computer program can controlling the cut depth in real-time.

In one embodiment, the computer program is configured to computing how multiple segments can be extracted from one donor cornea.

### Description of the figures

Exemplary embodiments of the invention are described using the figures. It is to be understood that these figures merely serve as examples of how the invention can be implemented and are in no way intended to be construed as limiting for the scope of the invention and the claims. Like features are indicated by like reference numerals along the figures. In the figures:
Figure 1 is a schematic view of a device for cutting an ophthalmic graft material according to a first example;
Figure 2 is a schematic view of a device for cutting an ophthalmic graft material according to a second example;
Figure 3 is a schematic view of a strip of ophthalmic graft material cut by a cutting tool according to a first example;
Figure 4 is a schematic view of a strip of ophthalmic graft material cut by a cutting tool according to a second example;
Figure 5 is a schematic view of a strip of ophthalmic graft material cut by a cutting tool according to a third example.

### Description of a detailed embodiment of the invention

Subsequently, some terms of the invention are described or defined.

By "ophthalmic graft material" it is meant a material suitable for serving as grafts in ocular/ophthalmic surgical procedures, particularly in procedures for reshaping the cornea surface. The ophthalmic graft material can be donor material from a donor or any other derived or synthesized graft material. The donor material is preferably a donor cornea. The donor material is for example an allogenic material derived from a donor (e.g. fresh, cryopreserved, or decellularized donor-derived corneal tissue). The synthesized graft substitutes could be a bioengineered material derived from biological sources or designed to mimic biological tissues (e.g. comprising collagen or collagen substitute), a synthetic material (e.g. comprising polymers such as polymethylmethacrylate), or a combination thereof.

The term "cutting tool" refers to a device, apparatus, or instrument configured to excise or cut an ophthalmic graft material. The cutting tool may be configured to perform cutting through one or more mechanisms such as mechanical (e.g., using a blade), thermal, optical, acoustic or a combination thereof (e.g., using a femtosecond lasers). The cutting tool may be manual (e.g. a manual trephine) or computer-controlled (e.g. a laser programmed to cut the spiral shaped strip).

By "strip" it is meant a continuous portion of ophthalmic graft material, having a strip length and a strip width. The strip is preferably used as implant for ophthalmic surgery, preferably for corneal surgery. The strip is preferably curved (e.g. arc shaped). The curved strip has preferably a constant curvature direction, i.e. the strip does not change its curvature direction like for a circular curvature (constant curvature) or a spiral shaped curvature (changing curvature with always the same curvature direction). The curvature of the strip can be mathematically expressed by the second derivation of the longitudinal axis of the strip. The curvature direction would correspond to the polarity of the second derivative, i.e. if the curvature is towards left or towards right.

By "spiral shaped strip" it is meant a strip that extends outward from a first end (also called centre of the spiral) of the ophthalmic graft material while turning around the first end at an increasing distance (also called spiral radius) from the first end. The first end of the spiral may be in a central region of a donor cornea, and the spiral shaped strip may extend towards a second end of the ophthalmic graft material which may be in a peripheral region of the donor cornea. For example, the spiral radius may increase linearly, exponentially as the spiral extends outward from its centre. The spiral radius may also increase sub-linearly, e.g. the rate of increase may diminish as the spiral extends outward from its centre. The distance between consecutive spiral turns may be a constant distance or a varying distance, e.g. the distance between consecutive spiral turns may increase or decrease as the spiral extends outward from its centre. For example, the spiral may be an Archimedean spiral, a logarithmic spiral, a Fermat's spiral, or a combination thereof. The spiral can be a single branch spiral, a double branch or a multi branch with two, three, four or more branches. The length of the spiral shaped strip corresponds to the length of the trajectory of a point moving inwardly along the spiral turns, towards the centre of the spiral (or outwardly from the centre of the spiral). The width of the strip may be constant (i.e., the spiral has a constant distance between consecutive spiral turns) or variable (i.e. the distance between consecutive spiral turns is varying, e.g. the strip tapers or expands outwardly from the centre of the spiral).

The inventive device 1 is configured to cut a strip from an ophthalmic graft material, preferably from a donor. The ophthalmic graft material is preferably a cornea material. The strip is preferably used in a surgical intervention in which the strip is implanted in an eye of a patient. The strip is preferably implanted in a pocket cut into the eye, preferably the cornea. The pocket is cut preferably by a Laser, preferably a femtosecond laser, or a manual trephine. The pocket has preferably an arced/curved shape, preferably the shape of a circular ring segment. The pocket is preferably arranged around the optical axis of the eye or around the center point of the eye.

The device 1 has preferably a first direction or z-direction. The first or z-direction shall also be referred to as vertical direction without limiting the invention with a use of the device 1 not aligned with the gravity of the earth. The device has preferably a defined first axis in the first direction. The device has 1 preferably a radial direction perpendicular to the first direction or z-direction. The radial direction shall also be referred to as horizontal direction. Two distinct radial directions shall span a horizontal plane. The device 1 has preferably a second direction or x-direction as a first radial direction and a third direction or y-direction as a second radial direction. The second direction and the third direction being perpendicular to each other.

The device 1 comprises a cutting tool 10 for cutting an ophthalmic graft material 2. The cutting tool 10 is configured to cut the ophthalmic graft material 2 such to extract a spiral shaped strip from the ophthalmic graft material 2. The cutting tool 10, 11 is preferably configured to cut the spiral shaped strip around the first axis of the device 1, preferably with the first axis of the device being the center point of the spiral. The cutting tool 10 is preferably configured to cut in the vertical direction, i.e. to separate with the vertically cut graft material arranged in a horizontal plane, preferably to separate neighboring turns of the spiral shaped strip. In some embodiments, it is also possible that the cutting tool 10 is configured to perform a horizontal cut. The horizontal cut is configured to separate the spiral shaped strip from deeper graft material 2 layers. The horizontal cut is preferably arranged in a defined cutting depth which defines the thickness of the spiral shaped strip. The cutting depth could be configurable by the user. The horizontal cut could follow a straight cut/direction or a curved direction/cut. The curved direction/cut could be dome shaped with highest cutting point at the first axis. The curved direction/cut could allow to have a constant thickness of the spiral shaped strip at different turns as the curved horizontal cut follows the surface of the cornea.

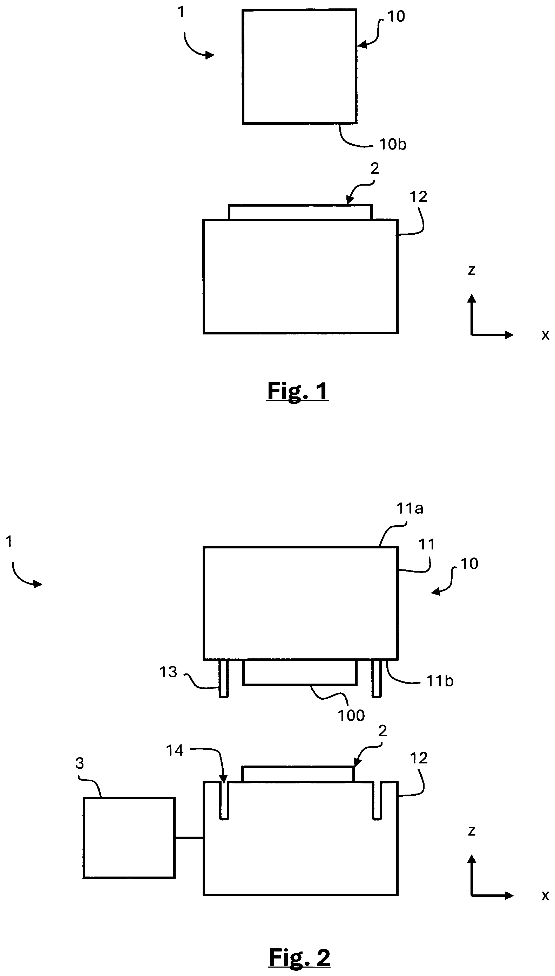

In one embodiment, the cutting tool 10 comprises a laser configured to cut out a spiral shaped strip from the ophthalmic graft material 2 as shown in Fig. 1. The laser 10 could be a femtosecond laser. In another embodiment, the cutting tool 10 comprises a blade 100 for cutting out the spiral shaped strip as shown in the example shown Fig. 2. In another embodiment, the cutting tool 10 comprises anything else configured to cut out a spiral shaped strip from the ophthalmic graft material 2.

In one embodiment, the device 1 comprises a graft holder 12 configured to hold the ophthalmic graft material 2 in a fixed position during the cutting with the cutting tool 10. The graft holder 12 comprises preferably a holding portion configured to hold the graft material 2. The holding portion is preferably arranged around the first axis of the device 1 or the graft holder 12, preferably with the first axis extending through the center of the holding portion. The holding portion has preferably a concave shape in order to position the ophthalmic graft material 2 better. However, it would also be possible to have flat or even convex holding portions. In the shown example, the ophthalmic graft material 2 is arranged on (the holding portion of) the graft holder 12. If the ophthalmic graft material 2 is from a donor, it has an optical axis extending through the center of the cornea and/or pupil of the eye of the donor. In this case, the optical axis of the ophthalmic graft material 2 is preferably arranged (substantially) coaxially to the first axis of the graft holder 12 or the device 1.

The graft holder 12 may be part of the device 1 or a separate component. The graft holder 12 is preferably removably connected to the cutting tool 10. This has the advantage that the ophthalmic graft material 2 can be placed better on the graft holder 12, when removed from the cutting tool 10. However, it would also be possible to have the graft holder 12 integrally fixed with the cutting tool 10. When the graft holder 12 is removably connectable to the cutting tool 10, preferably the device 1 has a mechanism for aligning the cutting tool 10 and the graft holder 12 in a defined relative position (to each other). The alignment mechanism aligns preferably at least the first axis of the graft holder 12 and the first axis of the cutting tool 10 (so that the first axes of the graft holder 12 and of the cutting tool 10 are coaxially arranged to the first axis of the device 1).

In the case of the example of Fig. 2, the alignment mechanism has a number of protrusions 13 (here rods) and a number of corresponding aligned 14 recesses (here holes) in the cutting tool 10 and the graft holder 12 so that a central axis (in the z-direction) of the cutting tool 10 and of the graft holder 12 are aligned, when the rods are inserted in the corresponding holes. The recesses 14 can be arranged all in one of the graft holder 12 and the cutting tool 10 and the protrusions 13 all in the other one of the two. In the example in Fig. 2, the protrusions 13 are arranged in the cutting tool 10 and the recesses 14 in the graft holder 12. However, it is also possible that the protrusions 13 are arranged in the graft holder 12 and the recesses 14 in the cutting tool 10. It would also be possible that one or more first protrusions 13 are arranged in the cutting tool 10 with their corresponding first recesses 14 in the graft holder 12, while one or more second protrusions 13 are arranged in the graft holder 12 with their corresponding first recesses 14 in the cutting tool 10. Many other alignment mechanisms are possible.

The graft holder 12 can comprise optionally a suction mechanism 3 may be configured to apply a negative pressure or suction between the ophthalmic graft material 2 and the (holding portion of the) graft holder 12. For example, the negative pressure may be applied to an inner lumen (not shown) of the graft holder 12 which is fluidly connected with the surface of the holding portion of the graft holder 12 configured to support the ophthalmic graft material 2. This first suction mechanism 3 allows to improve contact between the ophthalmic graft material 2 and the graft holder 12 and reduces the risk of movement of the ophthalmic graft material 2 during the cutting process. The suction might be generated manually (e.g. with a syringe or manual pump) or electrically (e.g. via an electrical pump).

In one embodiment, the cutting tool 10 may be a laser system, such as a femtosecond laser. The laser 10 is positioned to direct a laser beam along the vertical direction z to interact with the ophthalmic graft material 2 and excise the strip therefrom. The laser 10 could realize further a horizontal cut as described above in order to separate the spiral shaped strip from lower layers of the ophthalmic graft material 2. Especially for femtosecond lasers, the vertical and horizontal cutting line has to be distinguished from the trajectory of the laser 10 as the laser 10 cuts only at a certain point along the laser beam in order to cut 3D shapes and to cut deep layers of material. Thus, the laser trajectory in the radial direction does not necessarily mean that a vertical cut has been made where the laser beam has passed, if the laser 10 is configured to make an horizontal cut. The invention is not limited to the type of laser trajectory used. This laser trajectory can move along circles, spiral s) or straight lines or other trajectories.

In an alternative embodiment as shown in Fig. 2,the cutting tool 10 comprises a blade 100 for cutting the spiral shaped strip. The blade 100 has preferably the shape of the contours of the spiral shaped strip which should be cut out. This allows to move the blade 100 in the vertical direction to cut out the spiral shaped strip, similar to a punch or an ophthalmic trephine. Instead of the classic trephine with a circular blade, the cutting tool 10 would correspond to a trephine with a spirally formed blade 100 generating the spirally shaped cut in the graft material 2. More precisely, the cutting edge of the blade 100 has a spiral shape/itinerary/trajectory/curve. The spirally shaped blade 100 can thus be pushed/punched (along the first direction) into the graft material 2 in order to cut out the spiral shaped strip. The projection of the (cutting edge of the) blade 100 on a horizontal plane forms a spiral.

In one embodiment, the spiral shaped cutting edge of the blade 100 can be arranged in a horizontal and/or straight plane. In another embodiment, the spiral shaped cutting edge of the blade 100 is arranged in a curved plane, e.g. a dome shaped plane. The curved plane follows preferably an approximated shape of a cornea. The spirally shaped cutting edge of the blade 100 could have a continuously increasing distance from a horizontal plane when moving along the edge from the outside to the center point of the spirally shaped edge of the blade 100. This embodiment has the advantage that the cutting depth of the blade 100 in the graft material 2 from its surface has a constant thickness leading to a constant thickness of the spiral shaped strip.

The blade may be manufactured in many ways, e.g. by forming, stamping, and/or folding. The blade 100 is made of a material suitable for cutting an ophthalmic graft material 2 with high precision, for example a metal (e.g. stainless steel) or ceramic material or composites or alloys. The blade material might contain diamonds. The blade material can be a material capable of withstanding sterilization processes (e.g., by autoclaving or using ethylene oxide gas) and/or sharpening processes (e.g., mechanical sharpening and/or (electro)chemical etching). For example, the blade may be made of stainless steel.

The blade 100 may have a primary bevel angle. The primary bevel angle is preferably smaller than 20°, preferably smaller than 16°, preferably smaller than 15°, preferably smaller than 14°, preferably smaller than 13°, preferably smaller than 12°, preferably smaller than 11°. The blade 100 might have a secondary bevel angle close to the edge of the blade 100 smaller than the primary bevel angle (micro-bevel as common in Japanese knifes). The bevel angles might be symmetrical to the blade 100 or might be asymmetric.

In the shown example, the cutting tool 10 comprises a blade holder 11 having a proximal portion 11a and a distal portion 11b. The distal portion 11a faces towards the holding portion of the graft holder 12. The blade 100 is arranged on the distal portion 11b. The blade 100 extends preferably in the first or z direction from the blade holder 11. The blade 100 protrudes preferably from the distal portion 11b. The blade 100 can be reversibly (also called removably) or irreversibly attached to the blade holder 11.

However, the blade 100 can also be integrally formed with the blade holder 11 as a single piece. For example, the cutting tool 10 may be manufactured as a monolithic structure having a spiral shaped cross-section. One portion of the monolithic structure can be sharpened and serve as a cutting end, while another portion can remain unsharpened and serve as a holding end for manipulating the blade 100.

The device 1, the cutting tool 10, the blade 100 may be disposable, i.e. configured for a single-use. For example, the blade 100 could be disposable, while the rest of the device 1 is reusable. The disposable blade 100 could be inserted in the blade holder 11 each time a surgery or cutting is performed. It is also possible that the cutting tool 10 (with blade 100 and blade holder 11) is disposable. In this case, the blade 100 and the blade holder are forming preferably one piece. In this case, the graft holder 12 can be reusable. However, it is also possible that the complete device 1 with cutting tool 10 and graft holder 12 are disposable. On the other side, it is also possible that the complete device 1 including the blade 100 is reusable. All reusable components are configured for cleaning and sterilization after each use.

The blade holder 11 can be configured to hold blades 100 of different types, e.g. different spiral shapes and/or of different sizes. Different spiral shapes could have different strip widths. Different sizes could refer to the outer diameter of the blade 100.

The blade holder 11 might hold the blade 100 in a fixed position and the cutting could be realized by moving the cutting tool 10 (including blade holder 11 and blade 100) towards the graft holder 12, preferably along the first direction. However, it would also be possible that the blade holder 11 comprises a mechanism which moves the blade 100 in the first direction towards the graft holder 12 while the blade holder 11 remains still. The blade holder 11 could comprise a retraction mechanism for retracting the blade 100 in the blade holder 11.

The cutting tool 10 comprises preferably guide means which assures that the blade 100 during the cutting moves in the cutting direction, i.e. the first direction. In the shown embodiment, the guide means are realized with the alignment means described above. The alignment means allow the alignment of the graft holder 12 and the cutting tool 10 and the guidance of the cutting tool 10 along the first direction during the cutting. However, the guide means can be realized as a different means than the alignment means.

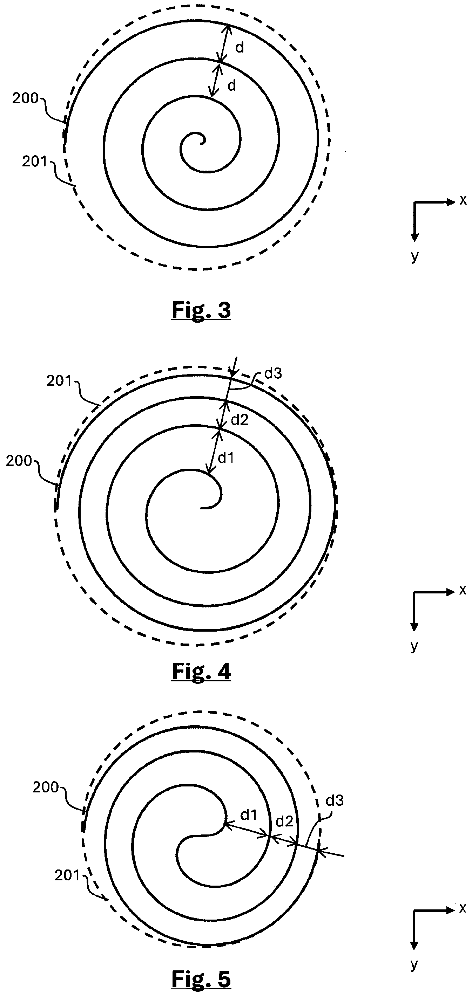

The cutting tool 10 is configured to cut spiral cutting lines 200 in the graft material 2 in order to obtain a spiral shaped strip. Preferably, the periphery of the graft material 2 is cut by a peripheral cutting line 201, wherein the spiral shaped cutting line 200 is arranged without the peripheral cutting line 201. The peripheral cutting line forms Figs. 3, 4 and 5 show exemplary cutting lines 200, 201 in a horizontal plane of the graft material 2 obtained by the cutting tool 10. The cutting line 200 is spiral shaped and leads to the cutting of a spiral shaped strip of the graft material 2. The peripheral cutting line 201 is preferably also realized by the cutting tool 1 and is configured to make a cut to delimit the outer perimeter of the cut graft material 2. The peripheral cutting line 201 is preferably circular. However, other shapes of the peripheral cutting line 201 are possible. The cutting line 200 and/or 201 can be realized by a blade 100 having a cross-sectional shape in the horizontal plane as the shape of the cutting line 200 and/or 201 described or shown. The cutting line 200 and/or 201 can also be realized by a laser.

The spiral cutting line 200 has any spiral shape which leads to a spiral shaped strip of the graft material 2. A spiral according to the invention is any shape which has more than one full turn. A turn of a spiral is a strip or cutting line which extends 360° around a center point of the spiral and has at the inner end of the turn a smaller radius (distance from the center point of the spiral) than at the outer end of the turn. The change of radius from the inner end of the turn/spiral towards the outer end of the turn/spiral is preferably steady/continuous. The steady change of the radius is in some spirals constant like for an Archimedean spiral or is in some spirals increasing (hyperbolic or logarithmic spiral) or is decreasing (Fermat's spiral). However, it is also possible that the change of radius from the inner end of the turn/spiral towards the outer end of the turn/spiral is unsteady, i.e. has jumps as would be the case in a rectangular spiral. The second or last turn of the spiral must not necessarily be a complete turn. When the spiral has more than one turn, the length of the spiral shaped strip is already longer than the length of a circular ring of the graft material. The spiral shaped cutting line 200 can also be squeezed/scaled in one dimension (e.g. in x or y dimension) so that the spiral has a kind of elliptic shape. The spiral shape is not limited to the shapes mentioned here, but any spiral type form with more than one full turn can be used.

Each spiral cutting line 200 has a diameter which may be comprised between 2 mm and 18 mm. These diameters are particularly suited for cutting ophthalmic graft materials. The blade diameter is the diameter of the smallest circle (depicted as a dashed line in Figs. 3, 4, and 5) that fully circumscribes the cutting line 200.

Fig. 3 schematically illustrates a spiral shaped cutting line 200 having constant distance d between consecutive spiral turns. This blade allows to cut a strip of ophthalmic graft material 2 having a constant width d. In the shown example, the spiral is an Archimedean spiral.

Fig. 4 schematically illustrates a spiral shaped cutting line 200 having a varying distance d1, d2, d3 between consecutive spiral turns. In this example, where the distance decreases as the number of turns increases (d1 >d2>d3). This cutting line 200 allows to cut a strip of ophthalmic graft material 2 that tapers outwardly from the centre of the spiral. In the shown example, the spiral is a Fermat's spiral

Fig. 5 schematically illustrates α spiral shaped cutting line 200 having a varying distance d1, d2, d3 between consecutive spiral turns. The distance decreases as the number of turns increases (d1>d2>d3) as in the example of fig. 4. In this example, the spiral is a double Fermat's spiral, i.e. it is made of two Fermat's spirals rotated of 180° with respect to each other.

The device 1 could be integrated in a robotic surgery system with an imaging system which is configured to align the cutting tool 10 with the ophthalmic graft material 2 and perform the cutting of the spiral strip automatically. In the robotic surgery system, the cutting tool 10 is preferably a laser. In this case, the same cutting tool 10 of the device 1 could be used for implanting the strip in the eye of the receiving patient. The imaging system could be OCT.

A software can be used to facilitate the cutting of the spiral shaped strip. The software would receive data one or more planned strip of one or more receiving patients and compute which parts of the spiral shaped strip would best suit each of those one or more planned strip. The planned strip is the required strip for a surgical eye intervention at the receiving patient, preferably a CAIRS surgery. The data of the one or more planned strips could comprise one or more of the shape of the strip, the length of the strip, the width of the strip, the thickness of the strip. Since each portion of the spiral shaped strip has a different curvature and radius, the angular length of a planned strip of a receiving patient is different to the angular length of the spiral shaped strip, especially for the smaller radiuses versus the center of the spiral. The software could calculate the correct length of each planned strip of the receiving patients for each portion of the spiral shaped strip. The software could decide further the size and/or type of spiral used for cutting the ophthalmic graft material 2 based on the data of the one or more planned strips. If the planned strips have different widths, the spiral design can be selected such that it has different widths of the strip and choose the corresponding parts of the spiral shaped strip to correspond best to the width of the planned strips.

The software could be integrated in a surgical planning software which receives imaging data from one or more eyes of the one or more patients. Based on these imaging data, the required planned strips can be defined. The data of the planned strips can then be used directly as explained above to optimize the process of cutting the spiral shaped strip into the strips which best correspond to the planned strips.

Such a software in combination with a device 1 with a laser as cutting tool 10 could automatically already cut the one or more strips for the receiving patients out of the spiral shaped strip. If the different planned strips require different thicknesses, the different parts of the spiral shaped strip can be cut out with different thickness based on the data from the planned strips.

The length of a spiral can be calculated as follows. Assume the spiral has an outer diameter D which marks the outer end point of the spiral and an inner diameter d which marks the inner starting point of the spiral. The outer diameter for a cornea is normally around 12 mm. The inner diameter d could be zero or more conservatively 2 mm. With a constant thickness t of an Archimedean spiral, the number of turns of the spiral can be calculated as $N = \frac{D - d}{2 \times t} .$ With t=2mm and d=2mm two and a half turns N=2,5 can be reached. This results in a total strip length of N times the average circumference of the spiral $L = \frac{π \times N \times \left(D+d\right)}{2} .$ For the above given example of a circular donor material of D=12mm, the strip length will be 55 mm. The strip length of a classic ring-shaped strip of a donor material of the same diameter D instead is only D*π= 38 mm. Thus, the inventive elliptical strip shape can provide for an increase of 45% of obtained strip length for the same donor material.

It should be understood that the present invention is not limited to the described embodiments and that variations can be applied without going outside of the scope of the claims.

## Claims

1. Device (1) for cutting an ophthalmic graft material (2), the device (1) comprising a cutting tool (10) for cutting the ophthalmic graft material (2), wherein the cutting tool (10) is configured to cut the ophthalmic graft material (2) such to extract a spiral shaped strip from the ophthalmic graft material (2).

2. Device (1) according to claim 1, wherein the strip has a constant width (d).

3. Device (1) according to claim 1, wherein the strip has a width (d) which gets smaller towards one end of the strip.

4. Device (1) according to any one of claims 1 to 3, wherein the cutting tool (10) is a laser with a functionality to automatically cut the spiral shaped strip out of the ophthalmic graft material (2).

5. Device (1) according to any one of claims 1 to 3, wherein the cutting tool (10) is a blade (100) having a spiral shape.

6. Device (1) according to claim 5 comprising a blade holder (11) having a proximal portion (11a) and a distal portion (11b), the blade (100) extending outwardly from the distal portion of the blade holder (11).

7. Device (1) according to claim 6, wherein the blade holder (11) comprises a cutting mechanism which moves the blade (100) out of the proximal portion in order to cut the strip out of the ophthalmic graft material (2).

8. Device (1) according to any one of claims 1 to 7, wherein the device (1) further comprises a graft holder (12) for supporting the ophthalmic graft material (2).

9. Device (1) according to claim 8, wherein the cutting tool (10) is configured to cooperate with the graft holder (12) during use.

10. The device (1) according to claim 8 or 9, the graft holder (12) comprising a suction mechanism (3) for fixing the ophthalmic graft material (2) on the graft holder (12) during the cutting of the ophthalmic graft material (2).

11. Device (1) according to any one of claims 7 to 12, the device (1) being a corneal trephine or a corneal punch.

12. Method for excising an ophthalmic graft material (2) using the device (1) according to any one of the preceding claims, the method comprising a step of cutting by means of the cutting tool (10) a strip of ophthalmic graft material (2), the strip being a spiral shaped strip.

13. A computer program comprising instructions configured, when executed on a processor, to perform the following steps:
receiving data of one or more planned strips of ophthalmic graft material for implantation in the eye or the eyes of one or more receiving patients;
computing for each of the one or more planned strips which part of a spiral shaped strip of ophthalmic graft material can be used.

14. Computer program according to the previous claim configured to calculate the correct length of the part the spiral shaped strip of ophthalmic graft material (2) based on the length of the corresponding strip for the receiving patient.

15. Computer program according to claim 13 and 14 configured to select the size and/or type of spiral used for cutting the ophthalmic graft material (2) based on the data of the one or more planned strips.
